# EUROPEAN PATENT APPLICATION

(11) **EP 4 497 414 A1**
(43) Date of publication of application: **29.01.2025**
(21) Application number: 23187135.1
(22) Date of filing: 23.07.2023
(51) Int. Cl.: A61F 2/24

(54) **ANNULOPLASTY DEVICE**

(71) Applicant: HVR Cardio Oy, 02600 Espoo (FI)
(72) Inventor: Keränen, Olli, 23741 Bjärred (SE)
(74) Representative: Invent Horizon IP

(57) **Abstract**

An annuloplasty device is disclosed comprising a first arcuate support, a second arcuate support, the first arcuate support comprises a first posterior bow, the second arcuate support comprises a second posterior bow, the first and second posterior bows are curved around a central axis and extend in respective first and second planes being essentially perpendicular to the central axis, wherein first and second posterior bows have respective first and second free ends, the first and second posterior bows extend from the respective free ends and are joined together at an apex, whereby the first posterior bow transitions to the second posterior bow over the apex, wherein the apex is configured to be arranged at a first commissure of the heart valve. A related method is also disclosed.

## Description

### Technical field

This invention pertains in general to the field of cardiac valve replacement and repair. More particularly the invention relates to an annuloplasty device for positioning at the heart valve annulus.

### Background

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure.

A problem with prior art annuloplasty implants is to achieve a reliable fixation at the annulus while at the same time accommodating with the movements of the beating heart. I.e. while it could be possible to provide for a reliably fixated implant in particular situations, there is often a tradeoff in attaining an optimal accommodation to the movements of the heart. This means that the implant to some extent impedes those natural movements, and/or possibly interacts with the surrounding anatomy at the implanted site in a detrimental fashion over longer time periods. An annuloplasty implant is intended to function for years and years, so it is critical with long term stability in this regard. A further problem of prior art devices follows from the aforementioned tendency to interfere with the anatomy, leading to reduced tolerances in accommodating variations in such anatomies of the heart valve. Thus, in addition to being associated with the mentioned problems, existing implants can have a limited degree of adaptability to varying anatomies. This in turn can require a more careful tailoring of the implant and/or procedure to the specific situation at hand, which increases the complexity and the time needed for the overall procedure. This entails a higher risk to the patient and it is thus a further problem of prior art devices.

The above problems may have dire consequences for the patient and the health care system. Patient risk is increased.

Hence, an improved annuloplasty device would be advantageous and in particular allowing for avoiding more of the above mentioned problems and compromises, and in particular allowing for improved accommodation to the valve anatomy for secure fixation, during the implantation phase, and for long-term functioning.

### Summary of the Invention

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect an annuloplasty device is provided comprising a first arcuate support, a second arcuate support, wherein the first and second arcuate supports are configured to be arranged on opposite sides of native heart valve leaflets of a heart valve, wherein the first arcuate support comprises a first posterior bow configured to be arranged on an atrial side of said heart valve and conform to a posterior aspect thereof, wherein the second arcuate support comprises a second posterior bow configured to be arranged on a ventricular side of said heart valve and conform to a posterior aspect thereof, wherein the first and second posterior bows are curved around a central axis and extend in respective first and second planes being essentially perpendicular to the central axis, wherein first and second posterior bows have respective first and second free ends, the first and second posterior bows extend from the respective free ends and are joined together at an apex, whereby the first posterior bow transitions to the second posterior bow over the apex, wherein the apex is configured to be arranged at a first commissure of the heart valve.

According to a second aspect an annuloplasty system is provided comprising an annuloplasty device according to the first aspect and a delivery device, wherein the delivery device is configured to connect to the first and second arcuate supports and force the first and second arcuate supports apart so that a separation distance (d) therebetween, along the central axis, is temporarily increased whereby the annuloplasty device assumes an expanded state, and wherein the delivery device is configured to be disconnected from the first and second arcuate supports so that the separation distance (d) is reduced and the annuloplasty device moves from the expanded state to a contracted state.

According to a third aspect a method of repairing a defective heart valve is provided, comprising positioning a first posterior bow of an annuloplasty device on an atrial side of said heart valve to conform to a posterior aspect thereof, positioning a second posterior bow of the annuloplasty device on a ventricular side of said heart valve to conform to a posterior aspect thereof, wherein the first and second posterior bows are curved around a central axis and extend in respective first and second planes being essentially perpendicular to the central axis, wherein first and second posterior bows have respective first and second free ends, the first and second posterior bows extend from the respective free ends and are joined together at an apex, whereby the first posterior bow transitions to the second posterior bow over the apex, the method comprising positioning the apex at a first commissure of the heart valve.

Further examples of the invention are defined in the dependent claims, wherein features for the second and subsequent aspects of the disclosure are as for the first aspect mutatis mutandis.

Some examples of the disclosure provide for facilitated fixation of an annuloplasty device to the heart valve.

Some examples of the disclosure provide for a more reliable fixation of an annuloplasty device to the heart valve.

Some examples of the disclosure provide for a less time-consuming fixation of an annuloplasty device to the heart valve.

Some examples of the disclosure provide for securing long-term functioning and position of an annuloplasty device.

Some examples of the disclosure provide for a less complex fixation procedure of an annuloplasty device.

Some examples of the disclosure provide for a reduced risk of damaging the anatomy of the heart such as the annulus or the valve leaflets.

Some examples of the disclosure provide for improved accommodation of an annuloplasty device to varying anatomies of the heart valve.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which examples of the invention are capable of will be apparent and elucidated from the following description of embodiments of the present invention, reference being made to the accompanying drawings, in which
Fig. 1a is a schematic illustration, in a top-down view, of an annuloplasty device according to one example;
Fig. 1b is a schematic illustration, in side view, of an annuloplasty device according to one example;
Figs. 2a-b are schematic illustrations of an annuloplasty device, positioned at opposite sides of the heart valve, according to one example;
Figs. 3a-c are schematic illustrations of an annuloplasty device, in a first side view (a), in a top-down view (b), and in a second side view (c), according to one example;
Fig. 4 is a schematic illustration, in a perspective view, of an annuloplasty device according one example;
Fig. 5 is a schematic illustration, in a side view, of an annuloplasty device according one example;
Figs. 6a-b are schematic illustrations, in side views, of an annuloplasty device according examples of the disclosure;
Figs. 7a-b are schematic illustrations, in side views, of an annuloplasty device according examples of the disclosure;
Fig. 8 is a schematic illustration, in side view, of an annuloplasty device according to one example;
Figs. 9a-b are schematic illustrations, in side views, of an annuloplasty device according examples of the disclosure;
Figs. 10a-c are schematic illustrations, in side views, of an annuloplasty device according examples of the disclosure;
Fig. 11a is a schematic illustration, in a side view, of an annuloplasty device according one example;
Fig. 11b is a schematic illustration, in a top-down, of an annuloplasty device according one example;
Figs. 12a-b are schematic illustrations, in side views, of an annuloplasty device according examples of the disclosure;
Fig. 13 is a schematic illustration, in side view, of an annuloplasty device according to one example;
Figs. 14a-b are schematic illustrations, in perspective views, of a detail of an annuloplasty device according examples of the disclosure;
Fig. 15a is a flow-chart of a method of repairing a defective heart valve according to one example; and
Fig. 15b is a flow-chart of a method of repairing a defective heart valve according to one example.

### Detailed description

Specific examples of the invention will now be described with reference to the accompanying drawings. This invention may, however, be embodied in many different forms and should not be construed as limited to the examples set forth herein; rather, these examples are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

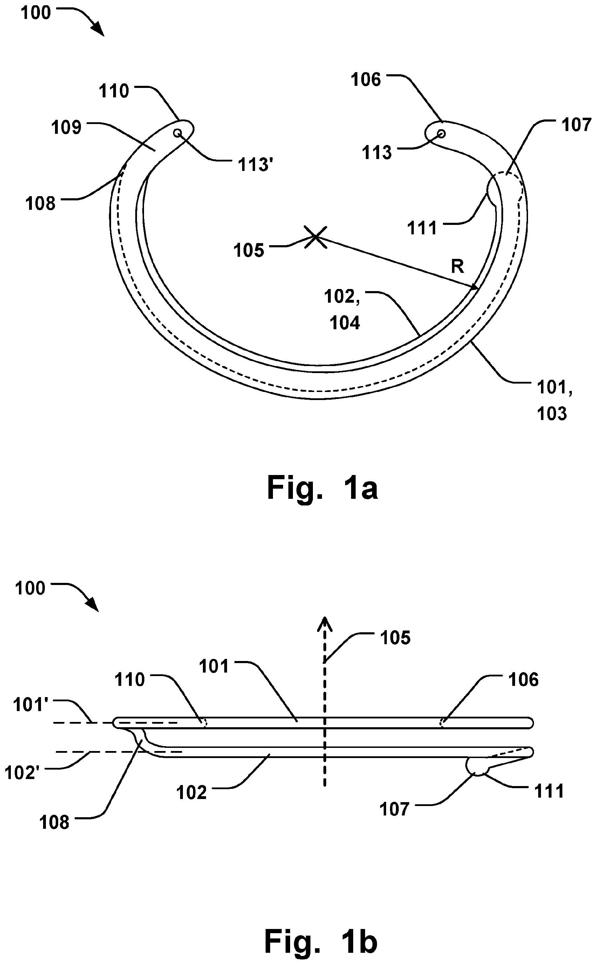

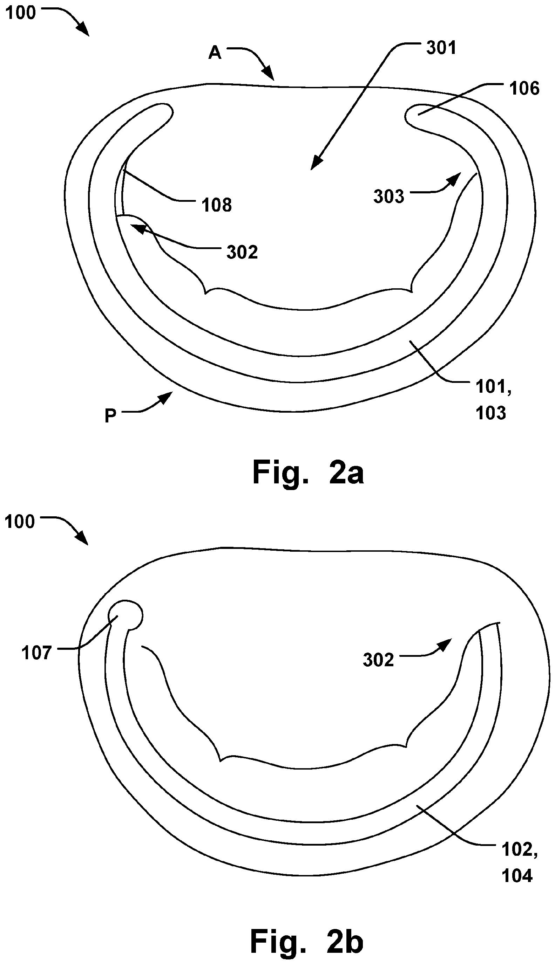

Figs. 1a-b schematically illustrate an example of an annuloplasty device 100 comprising a first arcuate support 101 and a second arcuate support 102, in a top-down view and in a side view, respectively. The first and second arcuate supports 101, 102, are configured to be arranged on opposite sides of native heart valve leaflets 301 of a heart valve. Figs. 2a-b show an example where the first arcuate support 101 is arranged on a first side of the heart valve, and the second arcuate support 102 is arranged on a second side of the heart valve, opposite the first side. The aforementioned first side may be an atrial side (Fig. 2a) of the heart valve, and the aforementioned second side may be a ventricular side (Fig. 2b) of the heart valve. The heart valve may be a mitral valve. Thus, the first arcuate support 101 comprises a first posterior bow 103 configured to be arranged on the atrial side of the heart valve. The first posterior bow 103 has a bow-shaped extension and is configured to conform to a posterior aspect (P) of the heart valve, i.e. along the posterior leaflet. The second arcuate support 102 comprises a second posterior bow 104 having a bow-shaped extension and is configured to be arranged on a ventricular side of the heart valve, and to conform to a posterior aspect (P) of the heart valve. Generally, the posterior side (P) of the heart valve may be seen as the bow shaped portion of the valve, extending from the anterior side (A), i.e. the anterior leaflet, which is less curved than the bow-shaped posterior side, see e.g. Fig. 2a.

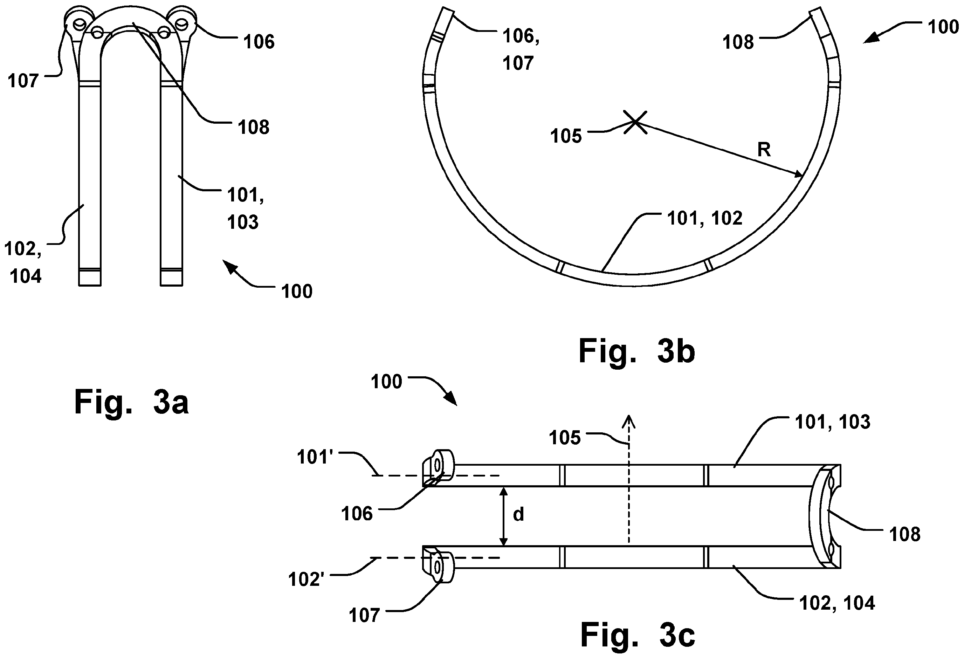

The first and second posterior bows 103, 104, are curved around a central axis 105 and extend in respective first and second planes 101', 102' being essentially perpendicular to the central axis 105. The top-down view of Fig. 1a shows an example of the first and second posterior bows 103, 104, being curved around a central axis 105, and the side view of Fig. 1b illustrates respective planes 101', 102', in which the first and second posterior bows 103, 104, extend. Figs. 3b and 3c show a further example of the first and second posterior bows 103, 104, being curved around a central axis 105, i.e. being in a curved configuration. The first and second posterior bows 103, 104, are arranged in the curved configuration at least when in a relaxed state of the material from which the device 100 is formed, i.e. free from outside forces acting upon the device 100.

The first and second posterior bows 103, 104, have respective first 106 and second 107 free ends, as schematically illustrated in e.g. Figs. 1a-b. The first and second free ends 106, 107, are configured to be arranged on the respective opposite sides of the heart valve leaflets 301, as exemplified in e.g. Figs. 2a-b. The first and second posterior bows 103, 104, extend from the respective free ends 106, 107, and are joined together at an apex 108, whereby the first posterior bow 103 transitions to the second posterior bow 104 over the apex 108, as schematically illustrated in Figs. 1a-b, 3a, and 3c. The apex 108 is configured to be arranged at a first commissure 302 of the heart valve, as exemplified in Fig. 2a. Although Fig. 2a shows an example where the apex 108 is arranged through the commissure denoted with reference numeral 302 it should be understood that the annuloplasty device 100 may be flipped over so that the apex 108 is arranged through the opposite commissure, denoted with reference numeral 303, in some examples.

The apex 108 of the annuloplasty device 100 may thus extend through the first commissure 302 when positioned and implanted at the heart valve, so that the first posterior bow 103 extend to the second posterior bow 104 through the first commissure 302 via the apex 108. The first and second posterior bows 103, 104, may be curved to follow along a correspondingly curved extension of the annulus of the heart valve, along the posterior side of the heart valve. The first and second posterior bows 103, 104, may thus assume the curved configuration also when in an implanted state. The annuloplasty device 100 may comprise a shape-memory material, so that the annuloplasty device 100 re-assumes the curved configuration after having been delivered from a catheter (not shown) to the target site at the heart valve, after having been temporarily restrained in an elongated configuration inside such catheter. The annuloplasty device 100 may comprise a shape memory material, such as NiTiNol, or another suitable biocompatible alloy that can be heat-set in defined shapes, i.e. in a defined relaxed shape in absence of outside acting forces, in a heat treatment procedure. Alternatively, the device 100 may maintain a curved configuration while being delivered to the target site, in which case it may be implanted at the target site for example by incision between the ribs or by opening the chest. The present disclosure, and the associated advantages described for the various examples, applies to both such variants of the device 100.

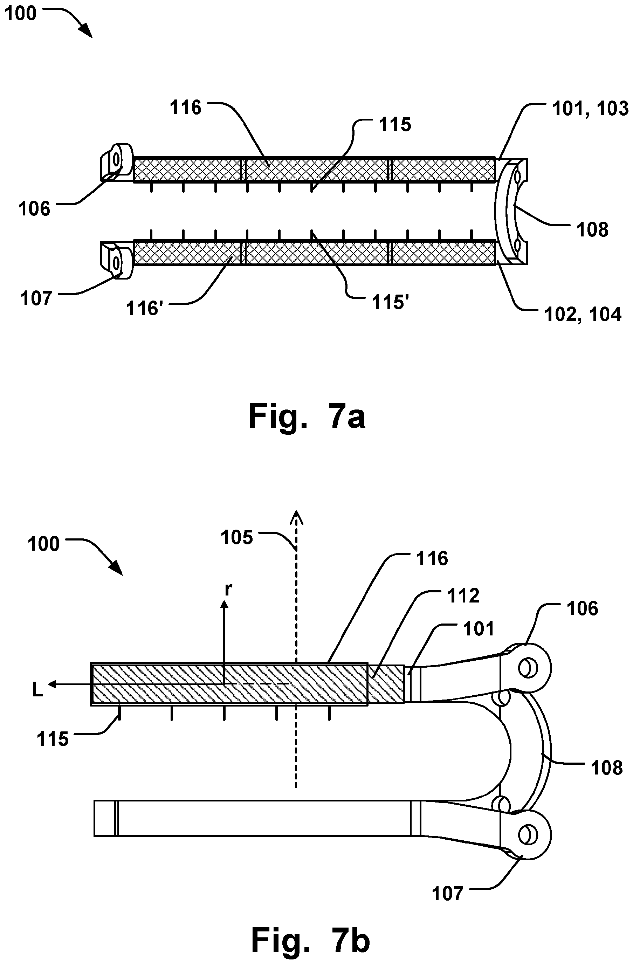

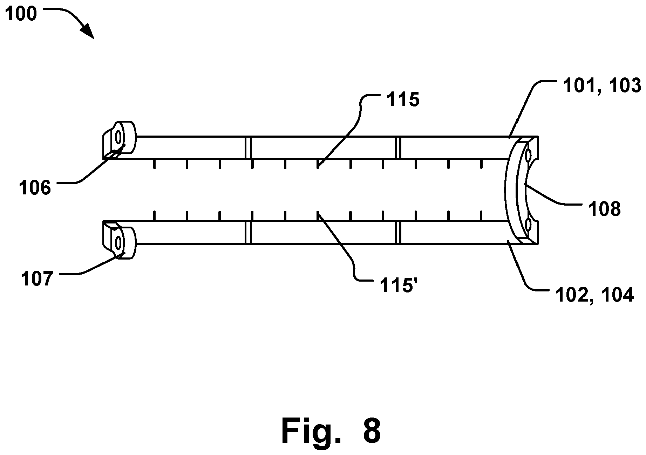

When implanted the annuloplasty device 100 may be retained or fixed at the heart valve by pinching the tissue of the valve leaflets 301, between the first and second posterior bows 103, 104, i.e. with forces acting parallel with the central axis 105. A separation distance (d) between the first and second posterior bows 103, 104, see e.g. Fig. 3c, may thus be chosen to provide for such pinching of the tissue when implanted. Alternatively, or in combination, the annuloplasty device 100 may be fixed at the heart valve by sutures (not shown), and/or retention units 115, 115', as described further below with reference to Figs. 7a-b and 8.

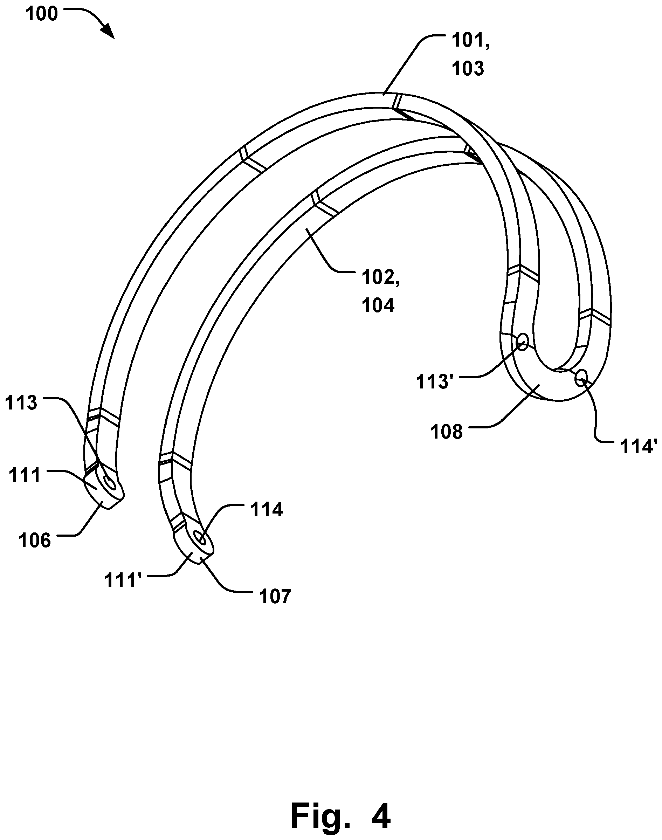

Fig. 4 is a schematic illustration of an example of the annuloplasty device 100 in a perspective view. The first and second posterior bows 103, 104, are connected via the apex 108 and extend in respective curved configurations therefrom towards free ends 106, 107. Having such first and second posterior bows 103, 104, connected at an apex 108 being configured to be arranged at a commissure 302 of the heart valve as described above with reference to e.g. Figs. 2a-b provides for a secure retention at the annulus along the posterior aspect (P) of the heart valve while minimizing interference with the anatomy along the anterior side (A), since the anterior side (A) can be free from an implanted structure on both the atrial side and the ventricular side. The anterior side (A) of the mitral valve is facing the aorta and the associated aortic valve. In some situations it has been observed that the mitral valve dilation can be advantageously reversed by reshaping of the annulus along the posterior side (P), as the dilation towards the anterior side (A) and the aorta outflow may be less pronounced in comparison. The annuloplasty device 100 is particularly advantageous in such situations as the annulus can be re-shaped to a modified shape along the first and second posterior bows 103, 104, where the modified shape of the tissue can be fixated by any of the above-mentioned mechanisms, e.g. by pinching of the valve tissue between the first and second posterior bows 103, 104, and/or retention units 115, 115', and/or sutures. Such annuloplasty can be done while avoiding having any implanted structure along the anterior side (A), which facilitates the natural movement of the heart valve and reduces the risk which otherwise may be associated with fixation of fastening units, such as sutures, along the anterior side (A). The risk of long-term tissue damage may thus be reduced, by avoiding any repeated interference along the anterior side (A) as the leaflets move with the beating heart. Reducing the length of the annuloplasty device 100 which needs to be secured provides also for a less time-consuming implantation procedure, as e.g. the number of sutures may be reduced.

Having the first and second posterior bows 103, 104, connected at an apex 108 being configured to be arranged at a commissure 302 of the heart valve as described above may also further optimize compliance with variations in the valve anatomy, since there will be a minimum of interference with the anatomy. For example, a greater variation the geometry and dynamics of the valve may be accommodated without interfering with the annuloplasty device 100. This may also provide for increasing the range of valve dimensions which are compliant with an annuloplasty device 100 of a particular size. Thus, during a particular implantation procedure, the annuloplasty device 100 provides for better adaptability and the tolerances for variations are increased.

The annuloplasty device 100 thus provides for an effective annuloplasty procedure with respect to obtaining the mentioned benefits for facilitating the implantation and improving long-term effects, while still allowing for a stable and secure fixation of the annulus in a modified shape where the valve leaflets can co-apt as intended.

The annuloplasty device 100 may be generally C-shaped in the first plane 101' or the second plane 102', as seen in e.g. the top-down views of Fig. 1a and 3b. The first arcuate support 101, or the first posterior bow 103 thereof, may extend in a general C-shape between the first free 106 end and the apex 108. The second arcuate support 102, or the second posterior bow 104 thereof, may extend in a general C-shape between the second free end 107 and the apex 108. The aforementioned general C-shape of the first and second arcuate supports 101, 102, may be shaped to follow the curvature of the annulus along posterior side of the heart valve. This provides for an effective accommodation to the heart valve anatomy of the shape of the annulus for a reliable fixation.

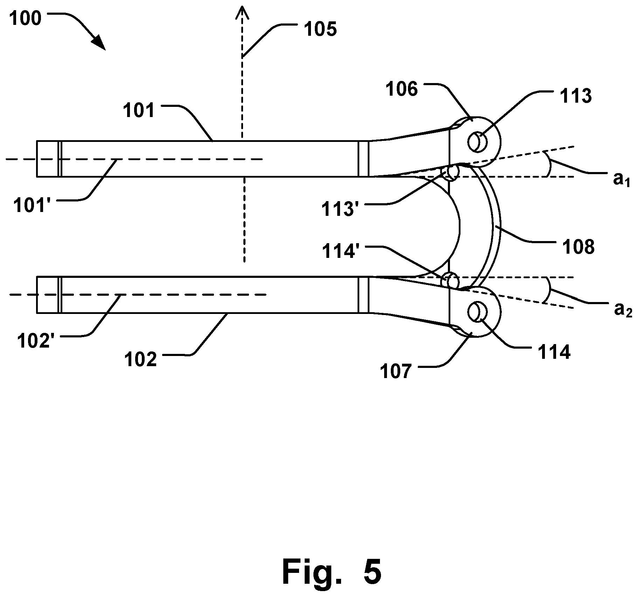

The apex 108 may extend with a curved shape between the first posterior bow 103 and the second posterior bow 104, as schematically illustrated in e.g. the perspective view of Fig. 4 or side view of Fig. 5. The apex 108 may thus provide a smooth transition between the first and second posterior bow 103, 104, which reduce the risk of tissue damage at the commissure 302, when the apex 108 is positioned through the commissure 302. Long-term functioning and reliability is thus improved.

The first arcuate support 101 may comprise an extension 109 from the apex 108 to a third free end 110, as schematically illustrated in the example of Fig. 1a. The extension 109 may provide for additional support for the tissue on e.g. the atrial side of the heart valve. The apex 108 may also extend with a curved shape between the first posterior bow 103 and the second posterior bow 104, as described above, eventhough comprising an extension 109 for support. The first arcuate support 101 may extend in a general C-shape between the first free end 106 and the opposite third free end 110, as shown in e.g. Fig. 1a.

The first posterior bow 103 may overlap the second posterior bow 104 in the first plane 101' or in the second plane 102'. In one example, the first posterior bow 103 may extend with substantially the same radius (R) from the central axis 105 as the second posterior bow 104, as shown in the example of Fig. 3b. This provides for an advantageous accommodation to the anatomy in some applications. The valve tissue may be effectively pinched between the first and second posterior bows 103, 104. In another example, the radius (R) to the central axis 105 may be different between the first and second posterior bows 103, 104, while still having an overlap. Fig. 1a shows an example where the aforementioned radius (R) is different, such as varying with a small amount, while still having an overlap between the first and second posterior bows 103, 104, in the top-down view, i.e. in the extension of the plane 101', 102'. In one example, the radius (R) of the second posterior bow 104, configured to be arranged in the ventricle, may be greater than the radius (R) of the first posterior bow 103, while still overlapping. This provides for an advantageous accommodation to the anatomy on the ventricular and atrial sides, while being able to pinch the tissue between the first and second posterior bows 103, 104.

The first free end 106 and/or the second free end 107 may comprise an at least partly spherical shape 111, 111' for non-traumatic tissue apposition, as schematically illustrated in e.g. Fig. 4. Such blunt free end 106, 107, provides for reducing the risk of damaging the surrounding tissue when positioning the annuloplasty device 100 at the heart valve.

The first free end 106 may extend with an angle (a₁) from the first plane 101', as schematically illustrated in the side view of Fig. 5. The first free end 106 may thus be angled upwards, away from the second support 102, in a direction towards the central axis 105. Having an angled first free end 106, when placing the first support 101 in the atrium, provides in some situations for a facilitated engagement of a delivery device (not shown) with the first free end 106, and thereby a facilitated manipulation and deployment of the annuloplasty device 100. Having an upward tilt of the first free end 106 may also further minimize the risk of having the tissue at the annulus exposed to the tip of the first free end 106. The tissue can instead be exposed to a smoother curvature of the first support 101.

Alternatively, or in addition, the second free end 107 may extend with an angle (a₂) from the second plane 102'. Fig. 5 shows an example where the second free end 107 is angled downwards, away from the first support 102, in a direction against the central axis 105. The central axis 105 extends vertically upwards in Fig. 5, from the second support 102 to the first support 101. Having a downward tilt of the second free 107 provides for a facilitate insertion of the second free end 107 into the commissure 302. The second free end 107 may thus more easily catch the commissure 302 when the second support 102 of the annuloplasty device 100 is to be inserted through the valve. The angle (a₁, a₂) may be in the range 5-15 degrees from the respective plane 101', 102', achieve the advantages described. An angle (a₁, a₂) of approximately 10 degrees from the respective plane 101', 102', provides for a particularly advantageous positioning of the annuloplasty device 100 while interference with the surrounding tissue.

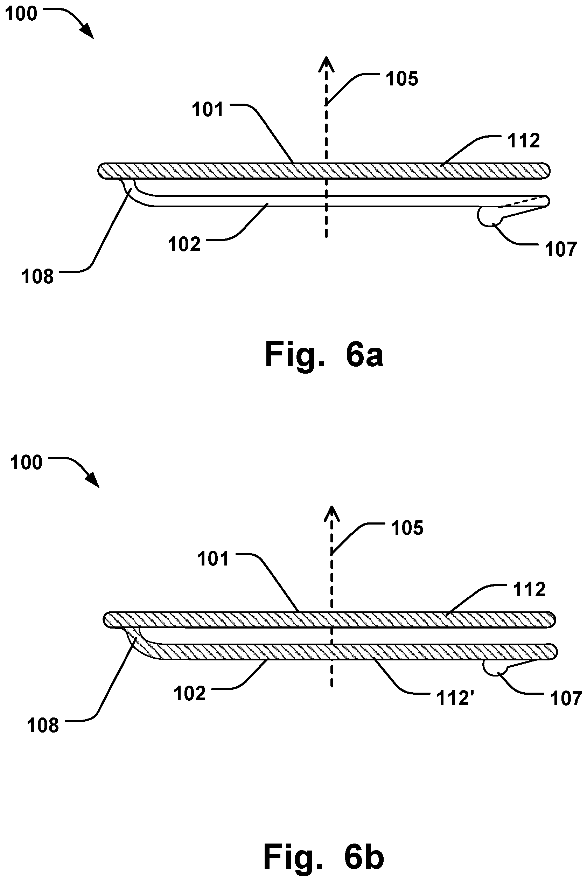

The annuloplasty device 100 may comprise a covering 112 arranged over at least part of the first arcuate support 101, as schematically illustrated in Fig. 6a. Alternatively, or in addition, the annuloplasty device 100 may comprise a covering 112' arranged over at least part of the second arcuate support 102, as schematically illustrated in Fig. 6b. The annuloplasty implant 100 may thus comprise an inner core of a shape memory material, i.e. the material from which the first and/or second arcuate support 101, 102, is formed, and an outer covering 112, 112', arranged radially outside the inner core material to cover at least part of the inner core. The outer covering 112, 112', may thus be resilient to conform to the inner core during movement of the shape memory material, e.g. if moving from an elongated delivery configuration inside a delivery catheter, to a curved configuration in the implanted state.

The covering 112, 112', may comprise a material having surface properties to promote endothelialization and the ingrowth of cells over the annuloplasty device 100. For example, the cover 112, 112', may have a surface which is more porous than the surface of the first- and/or second arcuate support 101, 102, which promotes the growth of cells over the annuloplasty device 100. The covering 112, 112', may comprise a mesh structure or any structure comprising an irregular, undulated, porous or otherwise non-flat surface to promote endothelialization. The covering 112, 112', may comprise a weave of a textile or a polymer.

The covering 112, 112', may be configured to be pierced by fastening units, such as sutures, clips etc, for fixing the annuloplasty device 100 to the tissue. The covering 112, 112', may be arranged around the entire length of the first- and second arcuate supports 101, 102.

The covering 112 may be attached to at least one fixation point 113, 113' arranged at the first arcuate support 101. Figs. 1a, 4 and 5 show examples of fixation points 113, 113', for a covering 112 (which has been omitted in the figures for clarity of presentation). Alternatively, or in addition, the covering 112' may be attached to at least one fixation point 114, 114' arranged at the second arcuate support 102, as further exemplified in Figs. 4 and 5. The fixation point 113, 113', 114, 114', may comprise a recess, protrusion, or through hole in the respective first and/or second arcuate support 101, 102.

The annuloplasty device 100 may comprise retention units 115 arranged along the first arcuate support 101, as schematically illustrated in Figs. 7a-b and 8. Alternatively, or in addition, the annuloplasty device 100 may comprise retention units 115' arranged along the second arcuate support 102, as further illustrated in Figs. 7a and 8. The retention units 115, 115', provides for a secure fixation of the annuloplasty device 100 at the annulus of the heart valve. The retention units 115, 115', may extend in opposite directions, along the central axis 105, from the respective first and second arcuate support 101, 102, in order to engage the tissue from opposite sides of the heart valve to create a strong retention force. The retention units 115, 115', may be deflected inside a delivery catheter (not shown) and exposed or expanded once the annuloplasty device 100 is ejected therefrom to engage the tissue.

The annuloplasty device 100 may comprise a stent 116, 116', arranged around at least a portion of the first and/or second arcuate support 101, 102, as schematically illustrated in Figs. 7a-b. The stent 116, 116', may comprise the respective retention units 115, 115'. The retention units 115, 115', are fixed in relation to the stent 116, 116', and the stent 116, 116', is fixed in relation to the first and/or second arcuate support 101, 102, on which the stent 116, 116', is arranged. Thus, having stents 116, 116', arranged around at least part of the first and/or second arcuate supports 101, 102, provides for anchoring the annuloplasty device 100 to the valve tissue with the retention units 115, 115'. The first and/or second arcuate supports 101, 102, are thus provided with a robust anchoring mechanism by utilizing a stent 116, 116', as an intermediate fixation structure for the retention units 115, 115'. This allows for an effective anchoring of the annuloplasty device 100 at the heart valve, due to the robust and reliable fixation mechanism provided by the stent 116, 116', and the retention units 115, 115', fixed thereto.

It should be understood that the annuloplasty device 100 may comprise a varying number of stents 116, 116', depending on the particular implant site of the annuloplasty device 100. The lattice or framework of the stent 116, 116', may be formed by laser cutting of a tube-shaped material, such as NiTinol or other bio compatible metal alloy and then pushed over the first and/or second arcuate supports 101, 102. The stent 116, 116', thus has a hollow interior to accommodate the first and/or second arcuate support 101, 102. The retention units 115, 115', may be formed from the material of the stent 116, 116', whereby the retention units 115, 115', are integrated with the stent 116, 116'.

The stent 116, 116', may be radially contractible along a radial direction (r), perpendicular to a longitudinal direction (L) of the stent 116, 116', so that the stent 116, 116', exerts a force on the first and/or second arcuate support 101, 102. The radial (R) and longitudinal direction (L) of the stent 116, 116', is schematically indicated in Fig. 7b. The stent 116, 116', may thus assume a fixed position in relation to the first and/or second arcuate support 101, 102, as the aforementioned force creates friction between the stent 116, 116', and the first and/or second arcuate support 101, 102. The framework of the stent 116, 116', may thus be cut to allow movement in the radial direction (r), i.e. allowing the support elements of the framework to move in relation to eachother, so that the diameter of the stent 116, 116', is variable.

The stent 116, 116', may be resiliently expandable in the radial direction (r) so that the stent 116, 116', may be expanded to a radially stretched state. The stent 116, 116', may then strive towards a contracted relaxed state with an inner diameter being less than the inner diameter in the radially stretched state. The inner diameter in the radially stretched state may be more or equal to an outer diameter of first and/or second arcuate support 101, 102. The stent 116, 116', may thus be positioned over the first and/or second arcuate support 101, 102, when in the radially stretched state. The stent 116, 116', will thus strive towards the contracted relaxed state with a reduced inner diameter, and accordingly exert the aforementioned force on the first and/or second arcuate support 101, 102. This provides for a facilitated fixation of the position of the stent 116, 116', in relation to the first and/or second arcuate support 101, 102.

The stent 116, 116', may exert a force onto the covering 112, 112', so that the covering 112, 112', is pinched between the stent 116, 116', and the first and/or second arcuate support 101, 102, as exemplified in the schematic illustration of Fig. 7b. Having a covering 112, 112', pinched between the stent 116, 116', and the first and/or second arcuate support 101, 102, provides for attaining a secure fixation of the position of the covering 112, 112', and the stent 116, 116', relative the first and/or second arcuate support 101, 102. The stent 116, 116', may thus strive towards an inner diameter which is smaller than an outer diameter of the covering 112, 112', when the latter is arranged around the first and/or second arcuate support 101, 102, so that a force is exerted radially inwards and pinches the covering 112, 112', against the outer surface of the first and/or second arcuate support 101, 102. In case the stent 116, 116', is formed from a temperature activated shape-memory material, the stent 116, 116', may increase the aforementioned force radially inwards as the stent 116, 116', is heated to the body temperature, which further increases the strength of the fixation of the stent 116, 116', relative the first and/or second arcuate support 101, 102.

Fig. 8 is a schematic illustration of an example where the retention units 115, 115', are directly fixed to the first and/or second arcuate support 101, 102, e.g. by welding, or an adhesive material. The retention units 115, 115', may also be provided by machining and forming of the material from which the first and/or second arcuate support 101, 102, is formed.

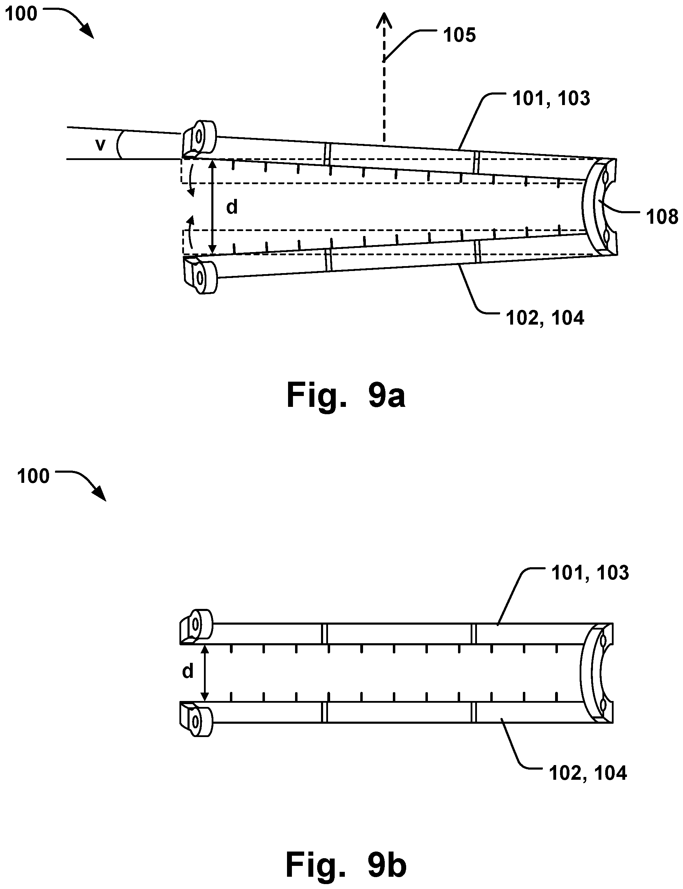

As mentioned above, the annuloplasty device 100 may comprise a shape memory material which has an elongated delivery configuration for advancement in a catheter (not shown), and a predefined arcuate shape of said shape memory material for positioning at an annulus of the heart valve, i.e. when ejected from the delivery catheter. Furthermore, the shape-memory material may be configured to be activated so that a separation distance (d) between the first and second arcuate supports 101, 102 is reduced to thereby pinch the valve tissue when placed at the opposite sides of the heart valve. Figs. 9a-b show an example where the separation distance (d) is reduced as the first and second arcuate supports 101, 102, move closer together when the shape-memory material of the first and/or second arcuate support 101, 102, is activated. I.e. the first and second arcuate supports 101, 102, move from the position shown in Fig. 9a to the position shown in Fig. 9b, from an expanded state to a contracted state. In one example the first and second arcuate supports 101, 102, may assume an angle (v) in the expanded state, as schematically illustrated in Fig. 9a, which is subsequently reduced in the contracted state, shown in Fig. 9b. It is also conceivable that the first and second arcuate supports 101, 102, remain essentially parallel in the expanded state, e.g. by expansion of the length of the apex 108 in the direction of the central axis 105, instead of increasing the angle (v). The length of the apex 108 in the direction of the central axis 105 may in such case decrease in the contracted state. In a further example the expanded state may be achieved by a combination of an increased angle (v) and an increase in said length of the apex 108.

The shape-memory material may be configured to be activated in response to setting a temperature of at least one of the first and second arcuate supports 101, 102, to an activation temperature. It is conceivable that the annuloplasty device 100 may be kept at a defined temperature, such as at a temperature below the activation temperature, while arranged in a delivery catheter (not shown). Subsequently, when the annuloplasty device 100 is exposed to the warm tissue, when being ejected from the delivery catheter, the activation temperature may be reached, so that the first and second arcuate supports 101, 102 are compressed towards eachother. The activation temperature may be close to the body temperature, e.g. just below the body temperature, so it takes longer time for the annuloplasty device 100 to reach the activation temperature when being subjected to the heat from the body. The annuloplasty device 100 may thus be placed in the curved or arcuate configuration at the opposite sides of the heart valve before the separation distance (d) is further reduced to pinch the valve tissue. This provides for a facilitated positioning of the annuloplasty device 100 at the opposite sides of the heart valve, in the expanded state, before the annuloplasty device 100 assumes the contracted state to pinch the valve tissue. The delivery catheter may in another example be utilized as a heat shield to delay the heating of the annuloplasty device 100 further, and/or a cooling medium may be circulated in the catheter to maintain the temperature of the annuloplasty device 100 below the activation temperature.

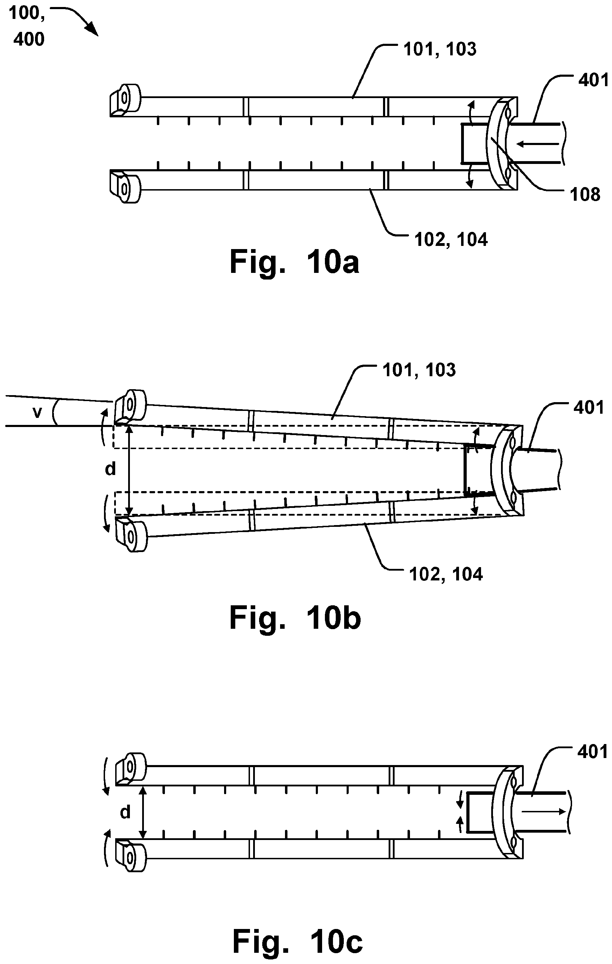

Figs. 10a-c show an annuloplasty system 400 comprising an annuloplasty device 100 as described above and a delivery device 401. The delivery device 401 is configured to connect to the first and second arcuate supports 101, 102, and force the first and second arcuate supports 101, 102, apart so that a separation distance (d) therebetween, along the central axis 105, is temporarily increased. Fig. 10a is a schematic illustration of a delivery device 401 engaging with the first and second arcuate supports 101, 102, before forcing the first and second arcuate supports 101, 102, further apart as schematically illustrated in Fig. 10b. The annuloplasty device 100 thus assumes an expanded state for facilitated positioning at the opposite sides of the heart valve. The delivery device 401 is configured to be disconnected from the first and second arcuate supports 101, 102, so that the separation distance (d) is reduced and the annuloplasty device 100 moves from the expanded state to a contracted state, as shown in Fig. 10c. Thus, as the annuloplasty device 100 has been placed in the correct position at the heart valve, the delivery device 401 is removed and the valve tissue is pinched first and second arcuate supports 101, 102, in the contracted state.

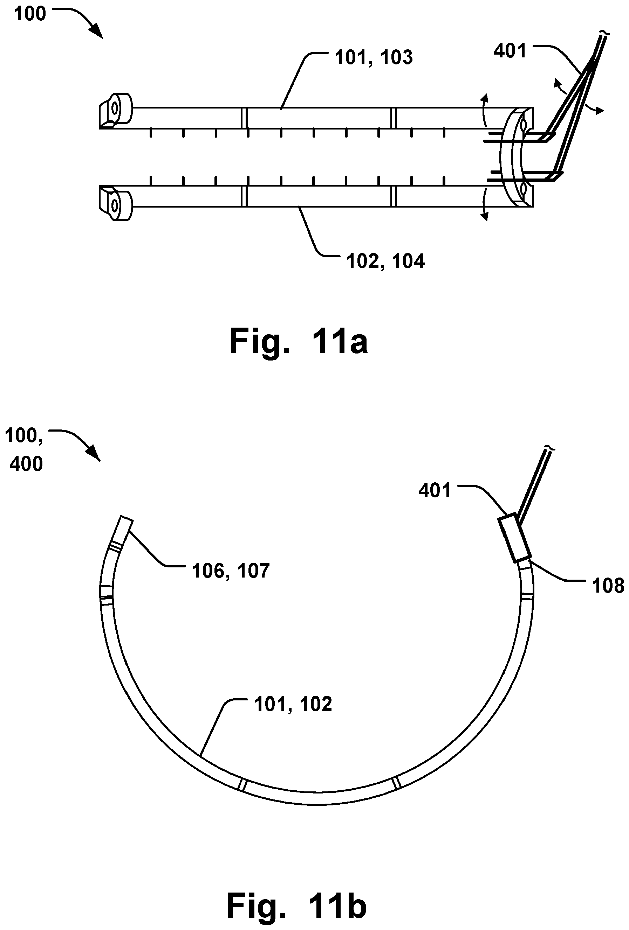

The delivery device 401 may push, pull, bend or otherwise move the first and second arcuate supports 101, 102, so that the expanded state is assumed. Figs. 10a-c shows an example where the delivery device 401 push the first and second arcuate supports 101, 102, apart. Fig. 11a show another example of a delivery device 401 engaging with the first and second arcuate supports 101, 102, adjacent the apex 108 (Fig. 11b) in order to bend the first and second arcuate supports 101, 102, apart, e.g. so that the angle (v), as seen in Fig. 10b, is increased, and/or so that the separation distance (d) is increased.

The first 101 and/or second arcuate support 102 may comprise a shape memory material, such as NiTiNol, or another suitable biocompatible alloy that can be heat-set in defined shapes, in a heat treatment procedure. The first 101 and/or second arcuate support 102 may comprise a polymer material in one example, having shape-memory properties. The shape-memory material may comprise a material having more than one phase, so that the shape of the arcuate supports 101, 102, may be actively varied as described above. The shape memory material can be conceived as any material that is able to change shape as desired, in response to outside interaction, for example with an energy source, such as providing heat and/or electromagnetic energy, that can be transferred to the annuloplasty device 100 to change its shape. It is also conceivable that the shape of the annuloplasty device 100 can be affected by direct mechanical manipulation of the curvature of the first 101 and/or second arcuate support 102, e.g. by transferring a force or torque to the annuloplasty device 100 via a delivery device. Via the various mentioned shape-affecting procedures the annuloplasty device 100 may assume an elongated delivery configuration for advancement in a catheter, an initial shape when positioned in a curved configuration along the annulus of the valve, and also an activated shape such as a contracted state where the separation distance (d) is reduced as described above for enhancing the strength of the fixation at an annulus of the heart valve.

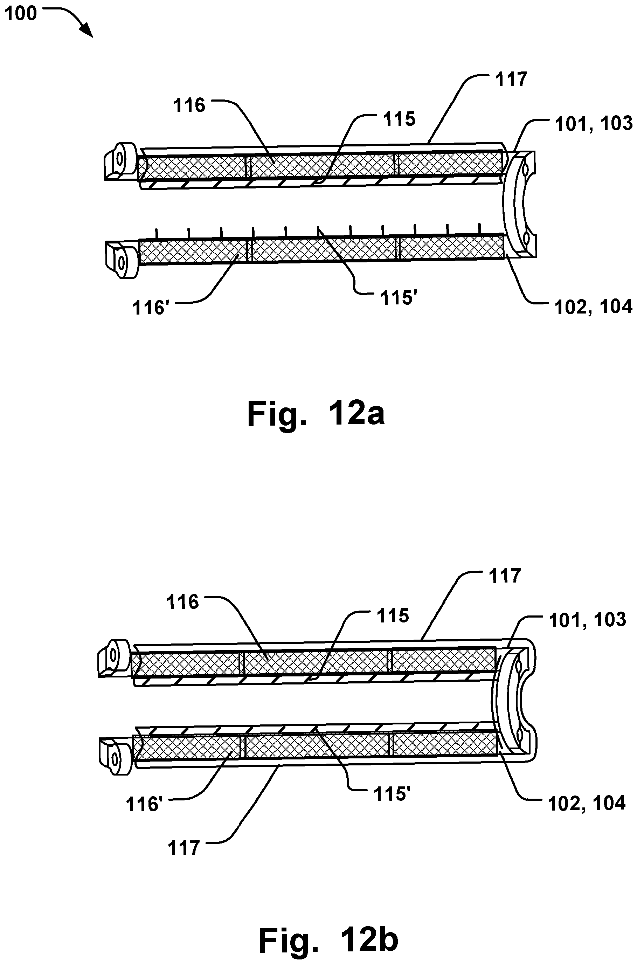

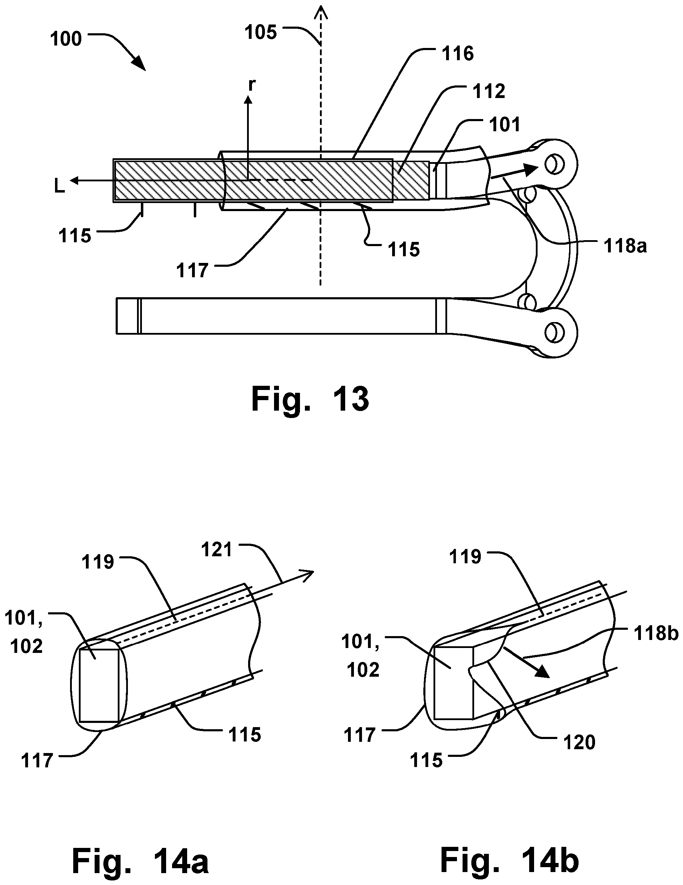

The annuloplasty device 100 may comprise a protective sheath 117 extending along at least part of the first arcuate support 101. Alternatively, or in addition, the protective sheath 117 may extend along at least part of the second arcuate support 102. The protective sheath 117 is configured to be removed from the annuloplasty device 100 when the annuloplasty device 100 has been arranged at the heart valve. Fig. 12a is a schematic illustration of a protective sheath 117 being arranged around the first arcuate support 101, and Fig. 12b is a schematic illustration of a protective sheath 117 being arranged around both the first arcuate support 101 and the second arcuate support 102. The protective sheath 117 provides for facilitated placement of the annuloplasty device 100 at the correct position at the heart valve since retention units 115 of the first and/or second arcuate support 101, 102, may be prevented from engaging with the tissue while the protective sheath 117 is arranged over the first and/or second arcuate support 101, 102. Thus, once the annuloplasty device 100 has been position at the opposite sides of the heart valve leaflets, the protective sheath 117 can be removed to expose the retention units 115 which may at that point pierce into the tissue and fixate the position of the annuloplasty device 100. The protective sheath 117 may be removed gradually so that the retention units 115 are gradually exposed and allowed to pierce into the tissue. The retention units 115 may be flexible and may assume a bent or angled position, as exemplified in e.g. Fig. 12a or Fig. 13, when surrounded by the protective sheath 117. The retention units 115 may expand outwards when the protective sheath 117 is removed. In one example, the direction in which the retention units 115 extend into the tissue when released from the protective sheath 117 may be affected by the direction in which the protective sheath 117 is pulled to be removed from the annuloplasty device 100. I.e. in case the retention units 115 are flexible they may be displaced towards the direction in which the protective sheath 117 is pulled upon removal, and thereby pierce into the tissue at an angle of such displacement, as further described in relation to Figs. 14a-b.

The protective sheath 117 may be removed from the annuloplasty device 100 by being pulled off the first and/or second arcuate support 101, 102, along the direction 118a in which the first and/or second arcuate support 101, 102, extends. This is exemplified in Fig. 13, where the protective sheath 117 is pulled in direction 118a as indicated by the arrow in Fig. 13. This may provide for gradually exposing the retention units 115 as illustrated in the example of Fig. 13. The retention units 115 may at that point transition from a compressed or angled position to an expanded position, which may correspond to a relaxed heat-set shape of the retention units 115, in case the retention units 115 are flexible. The protective sheath 117 may be made of a flexible material to be easily pulled off the annuloplasty device 100.

The protective sheath 117 may comprises a slit or a perforated portion 119 extending along a longitudinal direction 121 of the protective sheath 117, as schematically illustrated in Fig. 14a. The protective sheath 117 may in such case be configured to be removed from the annuloplasty device 100 along the slit or perforated portion 119, as exemplified in Fig. 14b. The protective sheath 117 may be torn along the perforated portion 119 and thus removed by being pulled in a direction such as indicated by arrow 118b in Fig. 14b. The protective sheath 117 may be bent off, or otherwise manipulated, to be removed from the annuloplasty device 100 in case of having a slit 119, i.e. a pre-made cut already provided along the longitudinal direction 121. The protective sheath 117 may in such case have a rigidity and structural integrity to stay in position around the first and/or second arcuate support 101, 102, as long as a force is not applied onto the protective sheath 117, e.g. along direction 118b which is non-parallel to the longitudinal direction 121, to peel off the protective sheath 117 from the annuloplasty device 100. Having a slit or a perforated portion 119 thus allows for removing the protective sheath 117 in several possible directions, which may facilitate removal of the protective sheath 117 and thereby expose the retention units 115. The protective sheath 117 may for example be pulled in a direction towards the center of the heart valve which thus may cause the retention units 115 to deflect in a direction towards the center of the heart valve upon removal of the protective sheath 117, as described above. The exposed retention units 115 may in such case be fastened into the annulus tissue in an advantageous direction to further improve the retention force of the annuloplasty device 100 at the heart valve. It is also conceivable that the protective sheath 117 may comprise other slits of perforations in order to facilitate removal of the protective sheath 117 from the annuloplasty device 100.

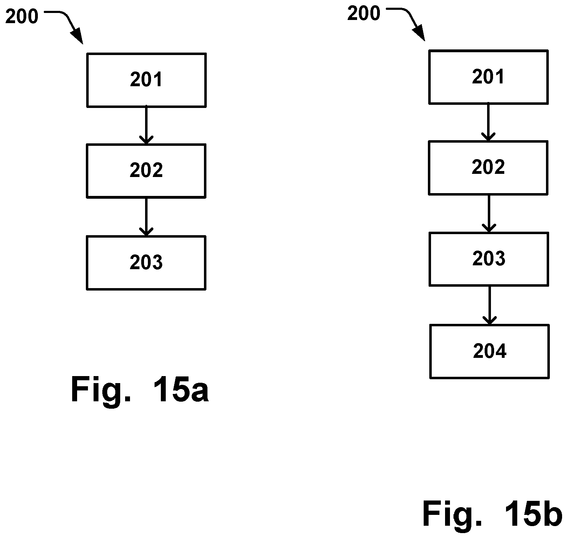

A method 200 of repairing a defective heart valve is provided. The method 200 is schematically illustrated in Fig. 15a, in conjunction with Figs. 1 - 14. The order in which the steps are described should not be construed as limiting, and it is conceivable that the order of the steps may be varied depending on the particular procedure. The method 200 comprises positioning 201 a first posterior bow 103 of an annuloplasty device 100 on an atrial side of the heart valve to conform to a posterior aspect thereof. The method 200 comprises positioning 202 a second posterior bow 104 of the annuloplasty device 100 on a ventricular side of the heart valve to conform to a posterior aspect thereof. The first and second posterior bows 103, 104, are curved around a central axis 105 and extend in respective first and second planes 101', 102' being essentially perpendicular to the central axis 105. The first and second posterior bows 103, 104, have respective first 106 and second 107 free ends. The first and second posterior bows 103, 104, extend from the respective free ends 106, 107, and are joined together at an apex 108. The first posterior bow 103 transitions to the second posterior bow 104 over the apex 108. The method 200 comprises positioning 203 the apex 108 at a first commissure 302 of the heart valve. The method 200 provides for the advantageous benefits as discussed above in relation to the annuloplasty device 100 and Figs. 1 - 14. The method 200 provides for a secure retention and implantation of the annuloplasty device 100 at the annulus along the posterior aspect (P) of the heart valve while minimizing interference with the anatomy along the anterior side (A). Improved accommodation to the surrounding anatomy at the heart valve is provided, and ultimately allowing for an improved fixation of the annuloplasty implant 100 and increased patient safety.

Fig. 15b is a further flowchart of a method 200 of repairing a defective heart valve. The first and second free ends 106, 107, may be arranged 204 adjacent a second commissure 303 opposite the first commissure 302 of the heart valve, as schematically illustrated in Figs. 2a-b.

The present invention has been described above with reference to specific embodiments. However, other embodiments than the above described are equally possible within the scope of the invention. The different features and steps of the invention may be combined in other combinations than those described. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. An annuloplasty device (100) comprising
a first arcuate support (101),
a second arcuate support (102),
wherein the first and second arcuate supports are configured to be arranged on opposite sides of native heart valve leaflets (301) of a heart valve,
wherein the first arcuate support comprises a first posterior bow (103) configured to be arranged on an atrial side of said heart valve and conform to a posterior aspect thereof,
wherein the second arcuate support comprises a second posterior bow (104) configured to be arranged on a ventricular side of said heart valve and conform to a posterior aspect thereof,
wherein the first and second posterior bows are curved around a central axis (105) and extend in respective first and second planes (101', 102') being essentially perpendicular to the central axis,
wherein first and second posterior bows have respective first (106) and second (107) free ends, the first and second posterior bows extend from the respective free ends and are joined together at an apex (108), whereby the first posterior bow transitions to the second posterior bow over the apex,
wherein the apex is configured to be arranged at a first commissure of the heart valve.

2. Annuloplasty device according to claim 1, wherein the annuloplasty device is generally C-shaped in the first or second plane.

3. Annuloplasty device according to claim 2, wherein the first arcuate support extends in a general C-shape between the first free end and the apex, and the second arcuate support extends in a general C-shape between the second free end and the apex.

4. Annuloplasty device according to any of claims 1 - 3, wherein the apex extends with a curved shape between the first posterior bow and the second posterior bow.

5. Annuloplasty device according to any of claims 1 - 3, wherein the first arcuate support comprises an extension (109) from the apex to a third free end (110).

6. Annuloplasty device according to claim 5, wherein first arcuate support extends in a general C-shape between the first free end and the opposite third free end.

7. Annuloplasty device according to any of claims 1-6, wherein the first posterior bow overlaps the second posterior bow in the first or second plane.

8. Annuloplasty device according to any of claims 1-7, wherein the first free end and/or the second free end comprise an at least partly spherical shape (111, 111') for non-traumatic tissue apposition.

9. Annuloplasty device according to any of claims 1-8, wherein the first free end extends with an angle (a₁) from the first plane, and/or wherein the second free end extends with an angle (a₂) from the second plane.

10. Annuloplasty device according to any of claims 1-9, comprising a covering (112, 112') arranged over the first and/or second arcuate support.

11. Annuloplasty device according to claim 12, wherein the covering is attached to at least one fixation point (113, 113') arranged at the first arcuate support, and/or wherein the covering is attached to at least one fixation point (114, 114') arranged at the second arcuate support.

12. Annuloplasty device according to any of claims 1 - 11, comprising retention units (115, 115') arranged along the first and/or second arcuate support.

13. Annuloplasty device according to claim 12, comprising a stent (116, 116') arranged around at least a portion of the first and/or second arcuate support, and wherein the stent comprises the retention units.

14. Annuloplasty device according to any of claims 1 - 13, wherein said annuloplasty device comprises a shape memory material and has an elongated delivery configuration for advancement in a catheter, and a predefined arcuate shape of said shape memory material for positioning at an annulus of said heart valve.

15. Annuloplasty device according to any of claims 1 - 14, wherein the first arcuate support and the second arcuate support are separated with a separation distance (d) along the central axis, and
wherein first and/or second arcuate support comprises a shape-memory material, wherein activation of the shape-memory material cause reduction of the separation distance so that the annuloplasty device assumes a contracted state.

16. Annuloplasty device according to any of claims 1 - 15, comprising a protective sheath (117) extending along at least part of the first arcuate support and/or along at least part of the second arcuate support, wherein the protective sheath is configured to be removed from the annuloplasty device when arranged at the heart valve.

17. Annuloplasty device according to claim 16, wherein the protective sheath comprises a slit or a perforated portion (119) extending along a longitudinal direction (121) of the protective sheath, whereby the protective sheath is configured to be removed from the annuloplasty device along the slit or perforated portion.

18. Annuloplasty system (400) comprising an annuloplasty device according to any of claims 1-17 and a delivery device (401), wherein the delivery device is configured to connect to the first and second arcuate supports and force the first and second arcuate supports apart so that a separation distance (d) therebetween, along the central axis, is temporarily increased whereby the annuloplasty device assumes an expanded state, and
wherein the delivery device is configured to be disconnected from the first and second arcuate supports so that the separation distance (d) is reduced and the annuloplasty device moves from the expanded state to a contracted state.

19. A method (200) of repairing a defective heart valve, comprising
positioning (201) a first posterior bow (103) of an annuloplasty device (100) on an atrial side of said heart valve to conform to a posterior aspect thereof,
positioning (202) a second posterior bow (104) of the annuloplasty device (100) on a ventricular side of said heart valve to conform to a posterior aspect thereof,
wherein the first and second posterior bows are curved around a central axis (105) and extend in respective first and second planes (101', 102') being essentially perpendicular to the central axis,
wherein first and second posterior bows have respective first (106) and second (107) free ends, the first and second posterior bows extend from the respective free ends and are joined together at an apex (108), whereby the first posterior bow transitions to the second posterior bow over the apex, the method comprising
positioning (203) the apex at a first commissure of the heart valve.

20. Method according to claim 19, wherein the first and second free ends are arranged (204) adjacent a second commissure opposite said first commissure of the heart valve.
